(19) Europäisches Patentamt
European Patent Office
Office européen des brevets

(11) Veröffentlichungsnummer: **0 153 646**
**B1**

(12) # EUROPÄISCHE PATENTSCHRIFT

(45) Veröffentlichungstag der Patentschrift: **01.07.87**

(51) Int. Cl.⁴: **C 07 C 143/828**

(21) Anmeldenummer: **85101442.3**

(22) Anmeldetag: **11.02.85**

(54) **Verfahren zur Herstellung von Alkenylsulfonylisocyanaten.**

(30) Priorität: **23.02.84 DE 3406476**

(43) Veröffentlichungstag der Anmeldung: **04.09.85 Patentblatt 85/36**

(45) Bekanntmachung des Hinweises auf die Patenterteilung: **01.07.87 Patentblatt 87/27**

(84) Benannte Vertragsstaaten: **CH DE FR GB LI**

(56) Entgegenhaltungen: **DE-B-1 230 016**

(73) Patentinhaber: **HOECHST AKTIENGESELLSCHAFT, Postfach 80 03 20, D-6230 Frankfurt am Main 80 (DE)**

(72) Erfinder: **Wegener, Peter, Dr., Am Eichkopf 4, D-6240 Königstein/Taunus (DE)**
Erfinder: **Riemenschneider, Wilhelm, Dr., Loreleistrasse 45, D-6230 Frankfurt am Main (DE)**
Erfinder: **Ulmschneider, Dieter, Dr., Im Flemetz 6, D-6240 Königstein/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1987

**Beschreibung**

Unter alkenylsulfonylisocyanaten sind hier Verbindungen mit der Struktureinheit

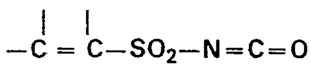

zu verstehen.

Sie sind hauptsächlich Zwischenprodukte auf verschiedenen Sachgebieten wie z.B. dem Pflanzenschutz-, dem Textilhilfsmittel-, dem Farbstoff- und dem Druckplattensektor.

Zur Herstellung der Alkenylsulfonylisocyanate ist eine Reihe verschiedener Methoden bekannt. Eine solche ist z. B. in der DE-PS 1 230 016 beschrieben. Danach werden Alkenylsulfonylisocyanate der allgemeinen Formel

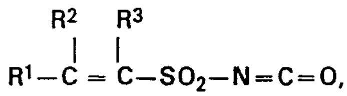

worin $R^1$, $R^2$ und $R^3$ = (unabhängig voneinander)

H- oder Halogenatome oder gegebenenfalls substituierte oder ringförmig verknüpfte Alkylgruppen hergestellt durch Umsetzung von 2-Chloralkylsulfonyliso-cyanaten der allgemeinen Formel

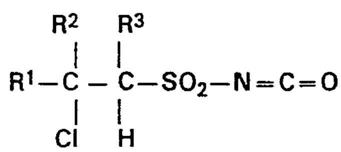

worin $R^1$, $R^2$ und $R^3$ die vorstehend angegebene Bedeutung besitzen,

mit tertiären aliphatischen Aminen vorzugsweise im Molverhältnis von etwa 1:1 bei Temperaturen zwischen etwa 10 und 80°C in Gegenwart eines reaktionsinerten Lösungs- oder Verdünnungsmittels.

Die Umsetzung, bei der aus den 2-Chloralkylsulfonyliso-cyanaten Chlorwasserstoff HCl abgespalten wird, verläuft nach der folgenden Reaktionsgleichung:

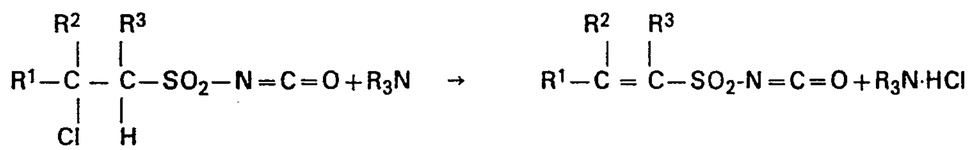

($R_1$, $R_2$, R3 besitzen die vorstehend angegebene Bedeutung,

R = alkyl).

Als reaktionsinerte Lösungs- oder Verdünnungsmittel kommen dabei solche Agentien in Frage, die weder mit dem eingesetzten noch mit dem bei der Reaktion gebildeten Isocyanat agieren und in denen das im Verlauf der Umsetzung infolge Bindung des abgespaltenen Chlorwasserstoffs entstehende Trialkylammoniumchlorid praktisch unlöslich ist. Namentlich genannt sind in der DE-PS Benzol, Diethyl-, Diisopropyl- sowie Di-n-butylether, Heptan, Methylcyclohexan, Tetrachlorkohlenstoff und Methylenchlorid. Sämtliche Beispiele - bis auf eines - wurden nach den angaben der DE-PS in Benzol durchgeführt; die mitgeteilten Ausbeuten an Alkenylsulfonylisocyanat liegen da zwischen etwa 50 und 90 % d.Th. Für das einzige Beispiel, in welchem nicht in Benzol gearbeitet wurde, ist Diethylether/Diisopropylether als Lösungs- oder Verdünnungsmittel angegeben (Beispiel 1); die mitgeteilte Ausbeute an Alkenylsulfonylisocyanat beträgt hier 70 % d.Th.

Das demnach nach der vorerwähnten DE-PS für die Durchführung der dort beschriebenen Reaktion weitaus bevorzugte Lösungs- oder Verdünnungsmittel Benzol ist jedoch toxikologisch nicht unbedenklich und daher unerwünscht. In den aliphatischen Ethern (Diethylether/Diisopropylether) gemäß Beispiel 1 wurde aber eine insbesondere für die Durchführung in größerem Maßstab nicht ganz ausreichende Ausbeute erzielt.

Es bestand daher die Aufgabe, das in der DE-PS 1 230 016 beschriebene Verfahren so zu modifizieren bzw. zu verbessern, daß ohne den Einsatz eines toxikologisch bedenklichen Lösungs- oder Verdünnungsmittels hohe - auch für die Durchführung in technischem Maßstab akzeptable - Ausbeuten an Alkenylsulfonylisocyanaten

erhalten werden.

Diese Aufgabe konnte erfindungsgemäß durch Verwendung des Methyl-tert.-butylethers als Lösungs- oder Verdünnungsmittel gelöst werden.

Erfindungsgegenstand ist daher ein Verfahren zur Herstellung von Alkenylsulfonylisocyanaten durch Umsetzung von 2-Chloralkylsulfonylisocyanaten mit tertiären aliphatischen Aminen in einem aliphatischen Ether als Lösungs- oder Verdünnungsmittel, das Verfahren ist dadurch gekennzeichnet, daß man als aliphatischen Ether Methyltert.-butylether verwendet.

Dadurch werden Ausbeuten an Alkenylsulfonylisocyanaten um 90 % d.Th. und gegebenenfalls auch darüber erreicht. Diese Ausbeuten liegen um mindestens etwa 10 % über den Ausbeuten, welche man im Fall des Einsatzes der nächstliegenden Lösungs- oder Verdünnungsmittel des Standes der Technik - nämlich des Diethyl-, Diisopropyl- und des Di-n-butylethers - erhält. Infolge des glatteren Reaktionsverlaufs in Methyl-tert.-butylether ist auch der Sumpfanteil bei der Enddestillation erheblich geringer als etwa in anderen aliphatischen Ethern. Gegenüber diesen anderen aliphatischen Ethern besitzt Methyl-tert.-butylether außerdem noch den erheblichen Vorteil des vollständigen Ausbleibens der Peroxidbildung.

Gegenüber dem in der DE-PS 1 230 016 als bevorzugtem Lösungsmittel für die Chlorwasserstoffaufspaltung aus 2-Chloralkylsulfonylisocyanaten offenbarten Benzol besitzt der Methyl-tert.-butylether vor allem den Vorteil der weitestgehenden toxikologischen Unbedenklichkeit.

Obwohl Methyl-tert.-butylether als Reaktionsmedium bei chemischen und pharmazeutischen Synthesen bekannt ist - vgl. z. B. Ullmann's Enzyklopädie der technischen Chemie, 4. Aufl. Bd. 16 (1978), Seite 302 - war es doch außerordentlich überraschend, daß gerade dieser Ether im Vergleich zu den anderen nächstkommenden aliphatischen Ethern bei der Herstellung von Alkenylsulfonylisocyanaten durch Chlorwasserstoffabspaltung aus 2-Chloralkylsulfonylisocyanaten ein so erheblich verbessertes Ergebnis liefert.

Die Durchführung des erfindungsgemäßen Verfahrens erfolgt im Prinzip in der gleichen Weise wie dies in der DE-PS 1 230 016 für die Herstellung von Alkenylsulfonylisocyanaten in anderen Lösungs- oder Verdünnungsmitteln beschrieben ist.

Bevorzugte Ausgangsstoffe sind daher auch im vorliegenden Fall die 2-Chloralkylsulfonylisocyanate der in der DE-PS 1 230 016 angegebenen allgemeinen Formel.

Beispielhafte Ausgangsstoffe sind 2-Chlorethylsulfonylisocyanat, 2-Chlorpropylsulfonylisocyanat, 2-Chlorbutylsulfonylisocyanat, 1-Methyl-2-chlorpropylsulfonylisocyanat, 2-Chlorcyclohexylsulfonylisocyanat und 2-Chlorhexylsulfonylisocyanat. Bevorzugt sind 2-Chlorpropylsulfonylisocyanat, 2-Chlorbutylsulfonylisocyanat und 2-Chlorpentylsulfonylisocyanat, insbesondere 2-Chlorpropylsulfonylisocyanat. Die genannten Chloralkylsulfonylisocyanate sind leicht zugänglich und können etwa aus den entsprechenden Olefinen durch radikalische Umsetzung mit Chlorsulfonylisocyanat hergestellt werden.

An tertiären aliphatischen Aminen kommen z. B. in Betracht: Trimethylamin, Triethylamin, Tripropylamin, Triisopropylamin, Tri-n-butylamin, Triisobutylamin etc. Bevorzugte Amine sind Trimethylamin und Triethylamin.

Die Ausgangsprodukte (2-Chloralkylsulfonylisocyanate und tertiäre aliphatische Amine) werden vorzugsweise im Molverhältnis von etwa 1:1 eingesetzt. Es kann jedoch auch ein Überschuß an 2-Chloralkylsulfonylisocyanat verwendet werden.

Für die Menge des einzusetzenden Lösungs- oder Verdünnungsmittels (Methyl-tert.-butylether) können kaum exakte Unter- und Obergrenzen angegeben werden. Nach unten ist die Lösungsmittelmenge dadurch begrenzt, daß der Ansatz noch einigermaßen gut rührbar sein muß; die obere Grenze wird praktisch durch Wirtschaftlichkeitserwägungen gesetzt.

Das Verfahren kann bereits bei Raumtemperatur durchgeführt werden. Vorzugsweise arbeitet man bei Temperaturen zwischen etwa 0 und 80°C, insbesondere zwischen etwa 0 und 20°C. Da die Reaktion exotherm verläuft, ist es mitunter zweckmäßig, während der Umsetzung das Reaktionsgefäß zu kühlen. Die hergestellten Alkenylsulfonylisocyanate lassen sich aus dem Reaktionsgemisch nach Absaugen oder Abzentrifugieren des gebildeten Trialkylammoniumchlorids und Verdampfen des bei der Umsetzung anwesenden Lösungs- oder Verdünnungsmittels durch Destillation in reiner Form gewinnen.

2Wegen des glatten Reaktionsverlaufes und - dadurch bedingt - der hohen Ausbeuten an Alkenylsulfonylisocyanat sowie des geringen Sumpfanteils bei der Enddestillation, und außerdem auch wegen des Unterbleibens der Peroxidbildung in dem Methyl-tert.-butylether, verbunden mit dessen weitestgehender toxikologischer Unbedenklichkeit, stellt die Erfindung einen erheblichen Vorteil und Fortschritt dar.

Das nachfolgende Beispiel soll der weiteren Erläuterung der Erfindung dienen. Auf dieses (Erfindungs-)Beispiel folgen noch 7 Vergleichsbeispiele (mit Diethyl-, Diisopropyl- und Di-n-butylether sowie auch noch mit zwei anderen aliphatischen Ethern (Dimethoxyethan und Tetrahydrofuran) und darüberhinaus auch noch mit Methylenchlorid und Acetonitril als Lösungs- oder Verdünnungsmitteln, woraus die Überlegenheit der Erfindung klar hervorgeht.

**(Erfindungs-)Beispiel:**

In einem 120 l-Stahlemaille-Rührkessel wurden 42 kg 2-Chlorpropylsulfonylisocyanat und 30 l Methyl-tert.butylether vorgelegt und 23,2 kg Triethylamin unter Kühlen so zudosiert, daß die Temperatur 20°C nicht überstieg. Nach 45 Stunden Rühren bei 15°C wurde das ausgefallene Triethylammonium-hydrochlorid über

eine Druckfilternutsche abgepreßt, mit 10 l Methyl-tert.-butylether nachgewaschen, die vereinigten Filtrate über einen Dünnschichtverdampfer bei 100° C Wandtemperatur und 16 Pa aufkonzentriert und das Konzentrat nochmals über einen Dünnschichtverdampfer destilliert. Dei 75° C/0,6 Pa gingen 32,5 kg Produkt über, das sich als 95 %iges Propenylsulfonylisocyanat (entsprechend einer Ausbeute von 91,6 % d.Th.) erwies. Der Sumpfanteil bei der Enddestillation betrug etwa 1,5 kg entsprechend etwa 5 % des Propenylsulfonylisocyanatgewichts.

Der Versuch wurde mit verschiedenen anderen Lösungsmitteln anstelle des Methyl-tert.-butylethers mehrmals wiederholt. Das Ergebnis ist in der nachstehenden Tabelle zusammengestellt.

**Tabelle**: Erfindungsbeispiel und Vergleichsbeispiele

| Bsp. | Lösungsmittel | Ausbeute an Propenyl-sulfonyliso-cyanat (% d.Th.) | Sumpfanteil bei der Enddestillation in % der Gesamtmenge an Propenylsulfonyl-isocyanat |
|---|---|---|---|
| Erf. | Methyl-tert.-butylether | 91 | ca. 5 |
| Vgl.-Bsp.: | | | |
| 1 | Diethylether | 78 | ca.15 |
| 2 | Diisopropylether | 70 | ca.20 |
| 3 | Di-n-butylether | 65 | ca. 20 |
| 4 | Dimethoxyethan | 82 | ca.15-20 |
| 5 | Tetrahydrofuran | 85 | ca.10-15 |
| 6 | Methylenchlorid | 78 | ca.20 |
| 7 | Acetonitril | 76 | ca.15-20 |

## Patentansprüche

1. Verfahren zur Herstellung von Alkenylsulfonylisoisocyanaten durch Umsetzung von 2-Chloralkylsulfonylisocyanaten mit tertiären aliphatischen Aminen in einem aliphatischen Ether als Lösungs- oder Verdünnungsmittel,
dadurch gekennzeichnet, daß man als aliphatischen Ether Methyl-tert.-butylether verwendet.

2. Verfahren nach anspruch 1, dadurch gekennzeichnet, daß man als 2-Chloralkylsulfonylisocyanate Verbindungen der allgemeinen Formel

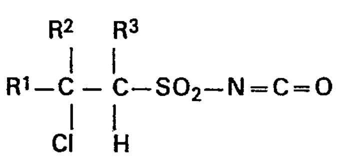

worin $R^1$, $R^2$ und R3 = (unabhängig voneinander)
H- oder Halogenatome oder gegebenenfalls substituierte oder ringförmig verknüpfte Alkylgruppen verwendet.

3. Verfahren nach Anspruch 2, dadurch gekennzeichnet, daß in der allgemeinen Formel für die 2-Chloralkylsulfonylisocyanate
$R^1 = C_1$-$C_3$-Alkyl, insbesondere nur Methyl und
$R^2 = R^3 = H$.

4. Verfahren nach einen oder mehreren der Ansprüche 1 bis 3, dadurch gekennzeichnet, daß man die Reaktionspartner im Molverhältnis von etwa 1:1 umsetzt.

5. Verfahren nach einem oder mehreren der Ansprüche 1 bis 4, dadurch gekennzeichnet, daß man die

Umsetzung bei Temperaturen zwischen etwa 0 und 80° C, insbesondere zwischen 0 und 20° C durchführt.

## Claims

1. A process for the preparation of alkenylsulfonyl isocyanatcs by reacting 2-chloroalkylsulfonyl isocyanates with tertiary aliphatic amines in an aliphatic ether as the solvent or diluent, characterized in that methyl tert.-butyl ether is used as the aliphatic ether.

2. The process as claimed in claim 1, characterized in that compounds of the general formula

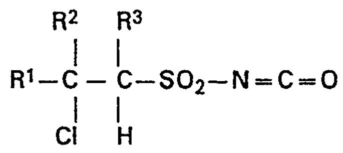

wherein $R^1$, $R^2$ and $R^3$ = (independently of one another) H or halogen atoms or optionally substituted or cyclized alkyl groups,
are used as the 2-chloroalkylsulfonyl isocyanates.

3. The process as claimed in claim 2, characterized in that
$R^1$ - $C_1$-$C_3$-alkyl, in particular only methyl, and
$R^2 = R^3 = H$
in the general formula for the 2-chloroalkylsulfonyl isocyanates.

4. The process as claimed in one or more or claims 1-3, characterized in that the reactants are reacted in a molar ratio of about 1:1.

5. The process as claimed in one or more of claims 1-4, characterized in that the reaction is carried out at temperatures of between about 0 and 80° C, in particular of between 0 and 20° C.

## Revendications

1. Procédé de préparation d'isocyanates d'alcénylsulfonyle par réaction d'isocyanates de 2-chloroalkylsulfonyle avec des amines aliphatiques tertiaires dans un éther aliphatique comme solvant ou diluant, procédé caractérisé en ce que l'on utilise l'éther méthyl-tert.-butylique comme éther aliphatique.

2. Procédé selon la revendication 1 caractérisé en ce que l'on part d'isocyanates de 2-chloroalkylsulfonyle de formule générale:

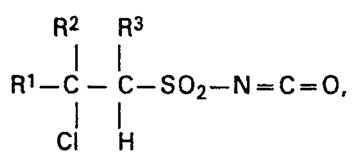

dans laquelle $R^1$, $R^2$ et $R^3$ désignent, indépendamment les uns des autres, l'hydrogène ou des atomes d'halogènes ou des alkyles éventuellement substitués ou reliés en formant un cycle.

3. Procédé selon la revendication 2 caractérisé en ce que dans la formule donnée $R^1$ est un alkyle en $C_1$ à $C_3$, en particulier le groupe méthyle et $R^2$ et $R^3$ sont chacun l'hydrogène.

4. Procédé selon l'une ou plusieurs des revendications 1 à 3, caractérisé en ce que l'on fait réagir les réactifs dans un rapport molaire d'environ 1.

5. Procédé selon l'une ou plusieurs des revendications 1 à 4, caractérisé en ce que l'on effectue la réaction à des températures d'environ 0 à 80° C, en particulier de 0 à 20° C.